# EUROPEAN PATENT SPECIFICATION

(11) **EP 2 505 152 B1**
(45) Date of publication and mention of the grant of the patent: **15.03.2017**
(21) Application number: 12162048.8
(22) Date of filing: 30.03.2012
(51) Int. Cl.: A61B 17/34

(54) **Access port and flexible sleeve with attached cord**
Zugangsport und flexible Hülse mit daran befestigtem Band
Orifice d'accès et manchon flexible avec cordon fixé

(30) Priority: 30.03.2011 US 201161469220 P; 13.09.2011 US 201113231123
(43) Date of publication of application: 03.10.2012
(73) Proprietor: Covidien LP, Mansfield, MA 02048 (US)
(72) Inventor: Rodrigues, Anibal Jr., Milford, CT 06460 (US)
(74) Representative: Soames, Candida Jane

(56) References cited:
- EP-A1- 2 238 933
- US-A1- 2003 078 478

## Description

### BACKGROUND

### Technical Field

The present disclosure relates generally to surgical apparatuses for use in minimally invasive surgical procedures, such as endoscopic and/or laparoscopic procedures, and more particularly, relates to a surgical apparatus that allows multiple surgical instruments to be inserted through a single incision.

### Description of Related Art

Today, many surgical procedures are performed through small incisions in the skin, as compared to large incisions that are typically required in traditional procedures, in an effort to reduce trauma to the patient and reduce the patient's recovery time. Generally, such procedures are referred to as "endoscopic", unless performed on the patient's abdomen, in which case the procedure is referred to as "laparoscopic." Throughout the present disclosure, the term "minimally invasive" should be understood to encompass both endoscopic and laparoscopic procedures.

During a typical minimally invasive procedure, surgical objects, such as endoscopes, graspers, staplers and forceps, are inserted into the patient's body through the incision in tissue. In general, prior to the introduction of the surgical object into the patient's body, insufflation gas is supplied to the target surgical site to enlarge its surrounding area and create a larger, more accessible work area. This is accomplished with a substantially fluid-tight seal that maintains the insufflation gas at a pressure sufficient to inflate the target surgical site.

It is desirable to insert instrumentation at the target surgical site while maintaining the pressure of the insufflation gas by using the substantially fluid-tight seal. Further, it is also desirable to permit multiple instruments of different dimensions operated through the substantially fluid-tight seal while maintaining the pressure of the insufflation gas.

The existing access devices in the prior art such as wound retractors are generally known for permitting operation of multiple instruments therethrough, but are also known for their drawbacks such as failure to prevent escape of insufflation gas when instruments of small dimensions are operated Document EP 2238933 A1, which forms the basis of the preamble of claim 1, discloses a flexible sleeve for placement in a tissue tract to allow access for a surgical instrument. The device comprises a cord contained within the wall of the sleeve to enable tightening of the said sleeve around an inserted surgical instrument.

Based on the above, a continuing need exists for an access device to provide enhanced sealing features.

### SUMMARY

Claim 1 discloses the invention and the preferred embodiments are described in the dependent claims. Disclosed herein is a surgical apparatus for positioning within a tissue tract accessing an underlying body cavity. The surgical apparatus includes a flexible sleeve defining a passage for reception of objects, and a cord attached to one end of the flexible sleeve.

In one embodiment, the passage of the flexible sleeve is configured to receive an access device therein. The passage defines a first diameter, the access device defines a second diameter, and the first diameter is greater than the second diameter. The cord is configured to secure the access device within the passage of the flexible sleeve.

In a certain embodiment, the cord is configured to reduce the diameter of the passage.

In a preferred embodiment, the cord is adapted to tie about an outer surface of the flexible sleeve.

### DESCRIPTION OF THE DRAWINGS

The above and other aspects, features, and advantages of the present disclosure will become more apparent in light of the following detailed description when taken in conjunction with the accompanying drawings in which:
**FIG. 1** is a front perspective view of a surgical apparatus in accordance with the principles of the present disclosure illustrating a surgical apparatus positioned relative to the tissue;
**FIG. 1A** is a side cross-sectional view of the surgical apparatus of FIG. 1;
**FIG. 2** is a front perspective view of the surgical apparatus of FIG. 1 in a tilted position prior to its insertion into the tissue;
**FIG. 3** is a front perspective view of the surgical apparatus of FIG. 1 disposed within the tissue prior to retraction;
**FIG. 4** is a front perspective view of the surgical apparatus of FIG. 1 disposed within the tissue in its retracted position;
**FIG. 5** is a side cross-sectional view of the surgical apparatus of FIG. 1 illustrating the surgical apparatus in its retracted position;
**FIG. 6** is a front perspective view of the surgical apparatus of FIG. 1 in its retracted position illustrating an access device positioned above the surgical apparatus;
**FIG. 7** is a front perspective view of the surgical apparatus of FIG. 6 illustrating the access device disposed within the surgical apparatus;
**FIG. 8** is a side cross-sectional view of the surgical apparatus of FIG. 7 illustrating the access device disposed within the surgical apparatus in a freestanding position; and
**FIG. 9** is a side cross-sectional view of the surgical apparatus of FIG. 8 illustrating the access device secured within the surgical apparatus.

### DETAILED DESCRIPTION

Particular embodiments of the present disclosure will be described herein with reference to the accompanying drawings. As shown in the drawings and as described throughout the following description, and as is traditional when referring to relative positioning on an object, the term "proximal" or "trailing" refers to the end of the apparatus that is closer to the user and the term "distal" or "leading" refers to the end of the apparatus that is farther from the user. In the following description, well-known functions or constructions are not described in detail to avoid obscuring the present disclosure in unnecessary detail.

One type of minimal invasive surgery described herein employs a device that facilitates multiple instrument access through a single incision. This is a minimally invasive surgical procedure, which permits a user to operate through a single entry point, typically the patient's navel. Additionally, the presently disclosed device may be used in a procedure where a naturally occurring orifice (e.g. vagina or anus) is the point of entry to the surgical site. The disclosed procedure involves insufflating the body cavity and positioning a portal member within, e.g., the navel of the patient. Instruments including an endoscope and additional instruments such as graspers, staplers, forceps or the like may be introduced within a portal member to carry out the surgical procedure. An example of such a surgical portal is disclosed in U.S. patent application Serial No. 12/244,024, Pub. No. US 2009/0093752 A1, filed October 2, 2008.

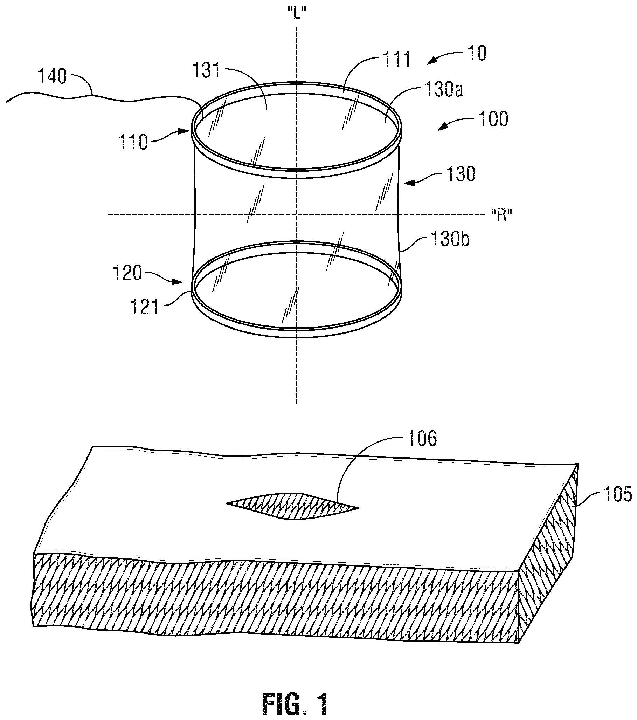

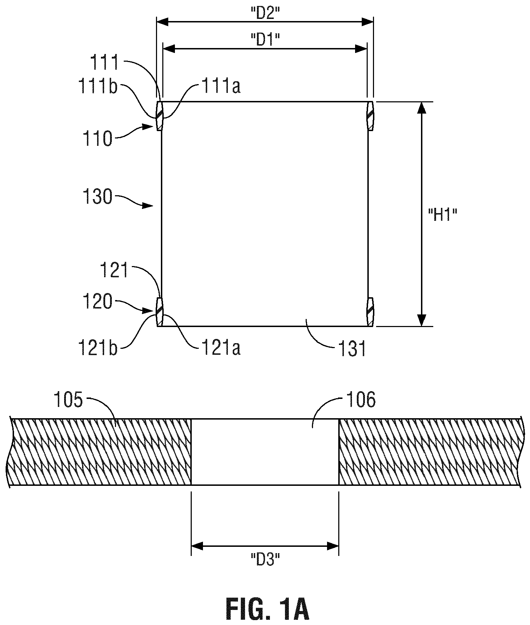

Referring now to the drawings, in which like reference numerals identify identical or substantially similar parts throughout the several views, **FIG. 1** illustrates a surgical apparatus **10** in accordance with the principles of the present disclosure. The surgical apparatus **10** is adapted for insertion in a tissue opening **106** within a tissue tract **105**, e.g., through the abdominal or peritoneal lining in connection with a laparoscopic surgical procedure. The surgical apparatus **10** will be described in greater detail hereinbelow.

As shown in **FIG. 1**, the surgical apparatus **10** includes a flexible sleeve 100 defining a generally cylindrical shape. However, it is contemplated that the sleeve 100 may define other configurations both prior and subsequent to insertion within the tissue tract **105**.

The sleeve 100 defines a longitudinal axis "**L**", a radial axis "R", and includes a proximal end 110 and a distal end 120 with a sheath or liner 130 extending therebetween. The sheath 130, in one embodiment, exhibits a generally cylindrical configuration. It is envisioned that the sheath 130 may exhibit other configurations. The sheath 130 defines an inner surface 130a and an outer surface 130b. The inner surface 130a defines a longitudinal passage 131 therein having a diameter of "D1" in its radial dimension as illustrated in FIG. 1A. In one embodiment, the radial dimension, "D1", is uniform along the length of the sheath 130. It is envisioned that the entire length of the sheath 130 is insertable into the tissue opening 106.

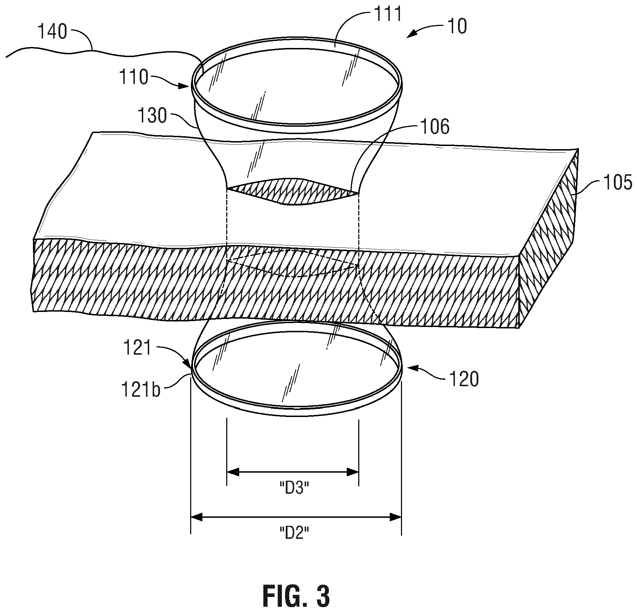

With additional reference to FIG. 1A, a ring member 111, 121 is mounted respectively at each end 110, 120 of the sleeve 100 in connection with the sheath 130. Each ring member includes an inner surface and an outer surface. For instance, the ring member 111 at the proximal end 110 includes an inner surface 111a and an outer surface 111b. The ring member 121 at the distal end 120 also includes an inner surface 121a and an outer surface 121b. The proximal ring member 111 is configured to be disposed exteriorly of the tissue opening 106, while the distal ring member 121 is configured to disposed interiorly with respect to the tissue opening 106. The inner surfaces 111a and 121a define an inner diameter "D1" identical to the diameter of the longitudinal passage 131. The outer surfaces 111b and 121b are configured to have an outer diameter "D2," which equals to or is greater than the radial dimension "D3" of the tissue opening 106. Due to their relatively large dimensions, the outer surfaces 111b and 112b inhibit the ring members 111 and 121 from travelling across the tissue opening 106 without user intervention. To insert any of the ring members (e.g. the distal ring member 121) through the tissue opening 106, the ring member 121 needs to be titled or biased to reduce its radial dimension to an extent (e.g. "D4") that is less than or equals to the radial dimension "D3" of the tissue opening 106. Once the distal ring member 121 is disposed interiorly of the tissue opening 106 as illustrated in FIG. 3, the distal ring member 121 returns to its normal, unbiased position. The radial dimension "D2" of the outer surface 121b of the distal ring member 121 facilitates anchoring the surgical apparatus 10 within the tissue tract 105.

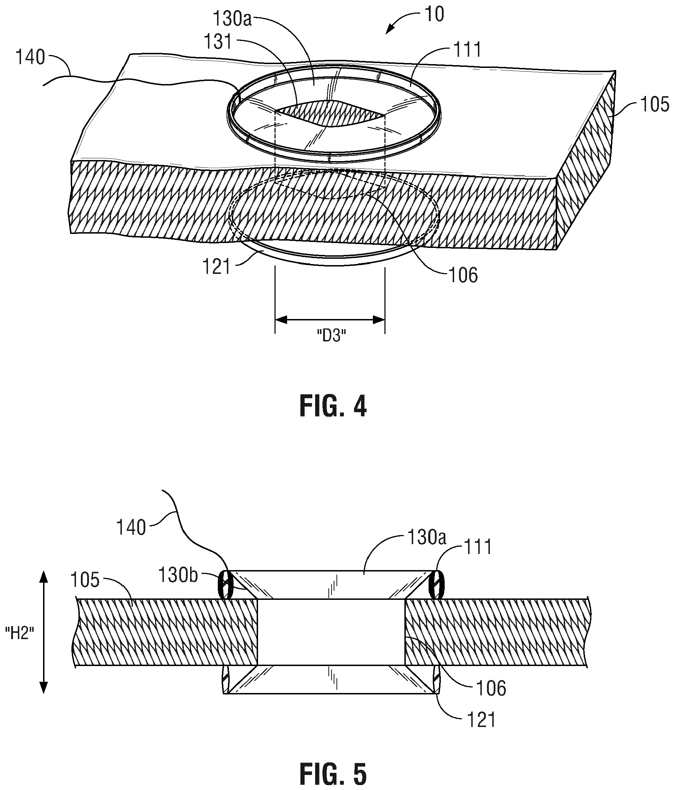

Each of the ring members (e.g. the proximal ring member 111) is configured to roll along the longitudinal length of the sheath 130 to approximate and retract the tissue tract 105, causing the height of the surgical apparatus 10 to approximate the thickness of the tissue tract 105, as illustrated in FIGS. 4 and 5. As the ring member 111 rolls along the longitudinal length of the sheath 130 in a distal direction, the sheath 130 is wrapped about the ring member 111 and rolled upon itself, and transitions from an unrolled or first state as illustrated in FIG. 1A to a rolled or second state as illustrated in FIGS. 4 and 5.

FIGS. 4 and 5 illustrate the surgical apparatus 10 in a retracted position at which the ring members 111 and 121 abut the tissue tract 105 thereby anchoring the surgical apparatus 10 with respect to the tissue tract 106. At the retracted position, the radial dimension of the longitudinal passage 131 is identical to that of the tissue opening 106 "D3." Also, at the retracted position, the sheath 130 forms a sealing relation with the tissue opening 106.

The surgical apparatus 10 defines a height from the distal end 120 to the proximal end 110. The height varies depending on the rolling state of the sheath 130. When the sheath 130 is unrolled, as illustrated in FIG. 1A, the surgical apparatus 10 has a maximum height of "H1." When the sheath 130 is rolled upon itself to an extent that the proximal end 110 meets the distal end 120, not shown, the surgical apparatus 10 has a minimum height. Therefore, as the ring member 111 rolls along the sheath 130 in a distal direction, the surgical apparatus 10 transitions from a maximum height to a minimum height. As illustrated in FIGS. 4 and 5, when the sheath 130 is rolled halfway, the surgical apparatus 10 has a height of "H2" which is less than the maximum height but greater than the minimum height.

The height of the surgical apparatus 10 can be adjusted to accommodate tissue tracts 105 of different thickness. In a situation when the maximum height of the surgical apparatus 10 readily fits in the tissue tract 105, the sheath 130 needs not be rolled upon. However, in situations in which the tissue tract **105** has a thickness, as illustrated in FIG. 5, that is less than the maximum height of the surgical apparatus 10, the ring member 111 may roll distally along the length of the sheath 130 to approximate the tissue tract 105, causing the height of the surgical apparatus 10 to approximate the thickness of the tissue tract 105, as illustrated in FIG. 5.

It is envisioned that a suture may be used to select, secure and maintain a desired height of the surgical apparatus 10. Other fastening means are also envisioned, including clips, snaps, or hooks for holding the surgical apparatus 10 at a desired height.

The surgical apparatus 10 further includes a cord 140 connected to the proximal end 110 as illustrated in FIG. 1. The cord 140 has a length sufficient to loop and tie circumferentially around the outer surface 130b of the surgical apparatus 10 to reduce the radial dimension of the longitudinal passage 131 of the surgical apparatus 10.

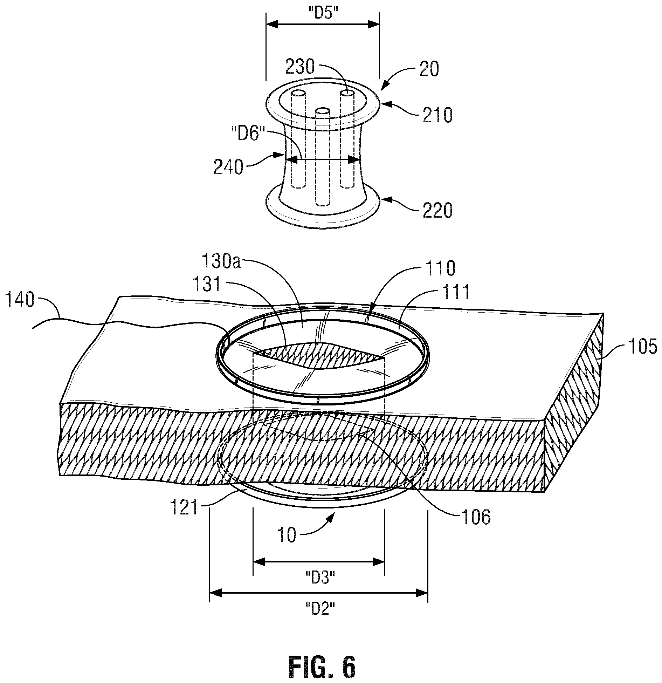

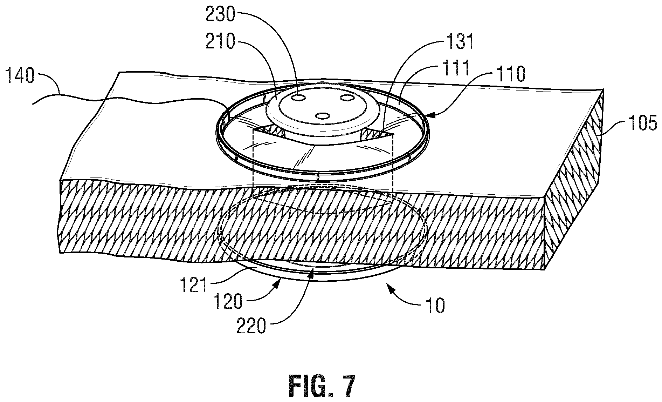

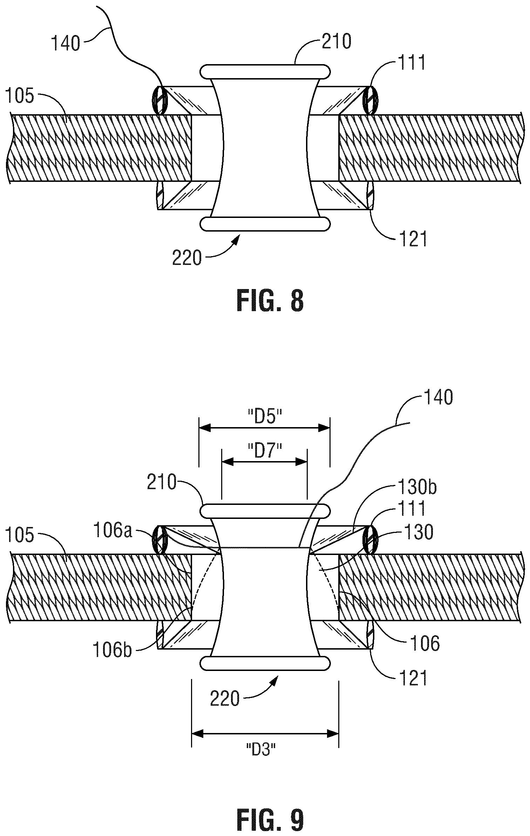

With reference to FIGS. 6 and 7, the longitudinal passage 131 of the surgical apparatus 10 is adapted to receive a portal member 20 such as that disclosed in U.S. patent application Serial No. 12/244,024, Pub. No. US 2009/0093752 A1, filed October 2, 2008. The portal member 20 defines at least one longitudinal passage 230 between its proximal end 210 and its distal end 220 for reception of a surgical object therethrough. The portal member 20 defines an hourglass configuration. In particular, with reference to FIG. 6, the portal member 20 defines a maximum radial dimension "D5" at its proximal end 210 and its distal end 220 which gradually becomes smaller towards the middle portion 240 where the minimum radial dimension "D6" is defined. The maximum radial dimension "D5" of the portal member 20 is less than the radial dimension "D3" of the longitudinal passage 131 when the surgical apparatus 10 is in its retracted position. Due to the small dimension of the portal member 20, if the portal member 20 were disposed in the surgical apparatus 10 in a freestanding position as illustrated in FIGS. 7 and 8, gravity would induce the portal member 20 to fall through the longitudinal passage 131 and into the patient's body cavity.

With additional reference to FIG. 9, the cord 140 is configured to loop and tie around the outer surface 130b of the surgical apparatus 10 immediately above the tissue opening 106, which, in turn, reduces the radial dimension of the longitudinal passage 131 of the surgical apparatus 10 that is positioned immediately above the tissue opening 106. By tying the cord 140 about the surgical apparatus 10, the longitudinal passage 131 at the upper side 106a of the tissue opening 106 is reduced to a radial dimension "D7" which is less than the maximum radial dimension "D5" of the portal member 20, thereby preventing the proximal end 210 of the portal member 20 from falling through. The longitudinal passage 131 at the upper side 106a of the tissue opening 106 forms a suspended relation with respect to the portal member 20, thereby securing the portal member 20 with respect to the surgical apparatus 10. Further, the longitudinal passage 131 at the upper side 106a of the tissue opening forms an air-tight or sealing relationship with the portal member 20. By contrast, the longitudinal passage 131 at the lower side 106b of the tissue opening 106 remains to have the same dimension "D3" as that of the tissue opening 106, and the sheath 130 at the lower side 106b of the tissue opening 106 continues to form a sealing relationship with the lower side 106b of the tissue opening 106.

The ring members 111 and 121 are made of a rigid or semi rigid material such as plastic or rubber. The sheath 130 is made of a flexible material that is also able to establish a sealing relation with the tissue tract 105 and is also able to form a sealing relation with surgical objects inserted within the longitudinal passage 131. The cord 140 is made of a flexible material, such as fiber or braid in the form of a thread or suture. The portal member 20 may be made from a semi-resilient, disposable, compressible and flexible type (e.g. rubber or sponge) material, for example, but not limited to, a suitable foam, gel material, or soft rubber having sufficient compliance to form a seal about one or more surgical objects, and also establish a sealing relation with the tissue tract **105** and with the surgical object. In one embodiment, the foam includes a polyisoprene material. The resilient nature of the portal member 20 provides an easy insertion and removal of the portal member 20 through the surgical apparatus 10.

In one embodiment, the cord 140 is an integrated part of the proximal end 110. The cord 140 is permanently attached to the proximal end 110 of the surgical apparatus 10 by glue, suture or by an overmolding process. In another embodiment, the cord 140 is detachably connected to the proximal end 110 of the surgical apparatus 10.

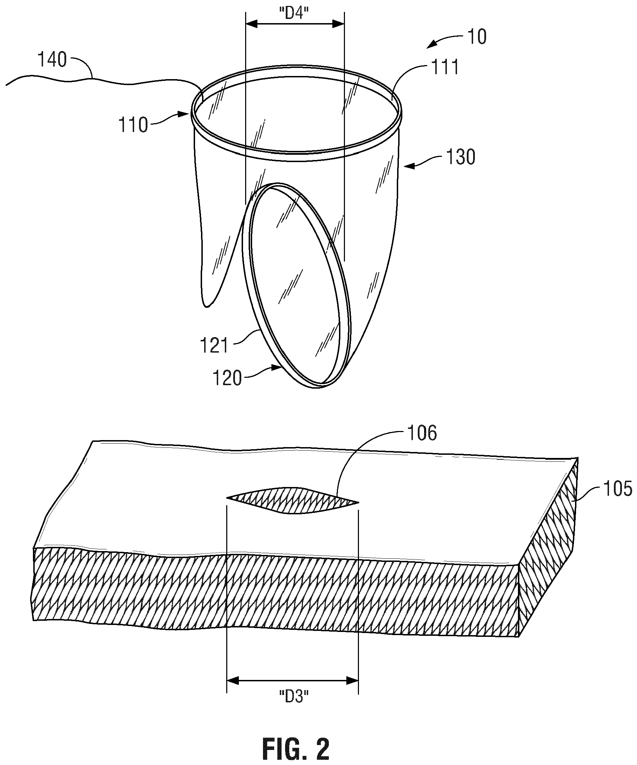

In operation, before insertion of the surgical apparatus 10 into the tissue tract 105, the surgeon first unrolls the sheath 130 to its unrolled state by rolling the proximal ring member 111 in a proximal direction along the length of the sheath 130, as illustrated in FIGS. 1 and 1A. Second, the surgeon tilts the distal end 120 of the surgical apparatus 10 as illustrated in FIG. 2, and introduces the distal end 120 of the surgical apparatus 10 into the tissue tract 105 through the tissue opening 106. Third, while the distal portion 120 is anchored interiorly with respect to the tissue tract 105 as illustrated in FIGS. 4 and 5, the surgeon retracts the tissue opening 106 by rolling the ring member 111 in a distal direction along length of the sheath 130 to create tension in the sheath 130. The same rolling step also causes the ring member 111 to approach the upper side of the tissue tract 105, which, in turn, reduces height of the surgical apparatus 10 to approximate the thickness of the tissue tract 105. Fourth, the surgeon introduces a portal member 20 into the longitudinal passage 131 of the surgical apparatus 10 as illustrated in FIGS. 6-8, and holds the proximal end 210 of the portal member 20 above the upper side of the tissue tract 105. Fifth, while holding the proximal end 210 of the portal member 20, the surgeon loops the cord 140 around the outer surface 130b of the sheath 130 to reduce the radial dimension of the longitudinal passage 131 of the surgical apparatus 10 at the upper side 106a of the tissue opening 106, so that the longitudinal passage 131 forms a sealing relation with respect to the portal member 20.

In use, the surgical apparatus 10 can sealingly engage instruments of various dimensions and maintain the insufflation pressure at the tissue opening 106. For instance, when a large instrument is desired to be operated through the tissue opening 106, it can be positioned directly within the longitudinal passage 131 and readily form a sealing relation with the surgical apparatus 10. When an instrument of a smaller dimension is desired to be operated through the tissue opening 106, the surgical apparatus 10 readily accommodates an intermediate access port (e.g. portal member 20) and forms a sealing relation therewith, and the intermediate access port is configured to sealingly receive instruments of small dimensions.

While several embodiments of the disclosure have been shown in the drawings and/or discussed herein, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Different embodiments of the disclosure may be combined with one another based on the particular needs of the patients to achieve optimal results of the surgical procedures. Those skilled in the art will envision other modifications within the scope of the claims appended hereto.

## Claims

1. A surgical apparatus for positioning within a tissue tract accessing an underlying body cavity comprising;
a flexible sleeve (100) defining a longitudinal axis (L), a proximal end (110), a distal end (120) and a passage (131) for reception of an object, the proximal end for location outside the body cavity, the distal end for insertion into the body cavity, the proximal and distal ends being open to provide access to the body cavity, and
a cord (140);
**characterized in that** one end of the cord is attached to the proximal end of the flexible sleeve (100), the remainder of the cord being unrestrained, and
the apparatus further comprises an access device (20) insertable into the flexible sleeve (100), whereby the cord (140) is adapted to be looped and tied around the outer surface of the said flexible sleeve, thereby reducing the diameter of the flexible sleeve to create a sealing relation between the said access device and the said sleeve.

2. The surgical apparatus according to claim 1 wherein the cord (140) is adapted to tie about an outer surface (130b) of the flexible sleeve (100).

3. The surgical apparatus according to claim 1, wherein the flexible sleeve (100) includes a sheath (130) in a generally cylindrical configuration.

4. The surgical apparatus according to any preceding claim, wherein the surgical apparatus defines an adjustable height.

5. The surgical apparatus according to any preceding claim, wherein looping and tying the cord (140) around the outer surface of the flexible sleeve (100) transitions the flexible sleeve from a first diameter to a second diameter.

6. The surgical apparatus according to claim 5, wherein the passage (131) defines the first diameter, the access device (20) defines the second diameter, and the first diameter is greater than the second diameter.

7. The surgical device according to claim 6 wherein the cord is configured to secure the access device (20) within the passage of the flexible sleeve (100).

8. The surgical apparatus according to any preceding claim, wherein the flexible sleeve (100) includes a proximal ring member (111) and a distal ring member (121) attached to each end thereof.

9. The surgical apparatus according to claim 8, wherein the distal ring member (121) is adapted for disposition interiorly of the tissue tract.

10. The surgical apparatus according to claim 8 or claim 9, wherein the proximal ring member (111) is adapted for disposition exteriorly of the tissue tract.

11. A surgical apparatus according to any one of claims 8 to 10, wherein
the ring members (111, 121) are arranged along the longitudinal axis (L);
the flexible sleeve (100) comprises a sheath (130) extending between the two ring members (111, 121) along the longitudinal axis (L); and
the cord (140) has two ends with one end attached to one ring member (111, 121), wherein the cord has sufficient length to loop and tie about the outer surface of the sheath transitioning the sheath from a first diameter to a second diameter.

12. The surgical apparatus according to claim 11, wherein the sheath (130) defines a passage (131) extending between the two ring members (111, 121) and configured to receive an object (20).

13. The surgical apparatus according to claim 11 or claim 12, wherein the sheath (130) defines a generally cylindrical configuration.

14. The surgical apparatus according to any of the preceding claims, wherein the cord (140) is configured to reduce the diameter of the passage (131) to form a sealing relation with the access device (20).

15. The surgical apparatus of any one of the preceding claims wherein the entire cord is disposed exteriorly to the body cavity.

16. The surgical apparatus of any one of the preceding claims wherein the entire cord is disposed exteriorly to the tissue tract.

## Patentansprüche

1. Chirurgische Vorrichtung zum Positionieren in einem Gewebetrakt für den Zugang zu einem darunter liegenden Körperhohlraum, umfassend:
eine flexible Hülse (100), die eine Längsachse (L), ein proximales Ende (110), ein distales Ende (120) und einen Durchgang (131) zur Aufnahme eines Gegenstandes definiert, wobei das proximale Ende zur Anordnung außerhalb des Körperhohlraums ist, das distale Ende zum Einsetzen in einen Körperhohlraum ist, wobei die proximalen und distalen Enden offen sind, um einen Zugang zu dem Körperhohlraum vorzusehen, und
eine Schnur (140);
**dadurch gekennzeichnet, dass** das eine Ende der Schnur an dem proximalen Ende der flexiblen Hülse (100) befestigt ist, wobei der restliche Teil der Schnur uneingeschränkt ist, und
die Vorrichtung ferner eine Zugangsvorrichtung (20) aufweist, die in die flexible Hülse (100) einführbar ist, wobei die Schnur (140) angepasst ist, um eine Außenfläche der flexiblen Hülse geschlungen und darum gebunden zu werden, wodurch der Durchmesser der flexiblen Hülse verringert wird, um ein abdichtendes Verhältnis zwischen der Zugangsvorrichtung und der Hülse zu erschaffen.

2. Chirurgische Vorrichtung gemäß Anspruch 1, wobei die Schnur (140) angepasst ist, um eine Außenfläche (130b) der flexiblen Hülse (100) gebunden zu werden.

3. Chirurgische Vorrichtung gemäß Anspruch 1, wobei die flexible Hülse (100) einen Mantel (130) in einer im Allgemeinen zylinderförmigen Konfiguration aufweist.

4. Chirurgische Vorrichtung gemäß einem der vorhergehenden Ansprüche, wobei die chirurgische Vorrichtung eine einstellbare Höhe definiert.

5. Chirurgische Vorrichtung gemäß einem der vorhergehenden Ansprüche, wobei das Umschlingen und das Binden der Schnur (140) um die Außenfläche der flexiblen Hülse (100) die flexible Hülse von einem ersten Durchmesser zu einem zweiten Durchmesser übergehen lässt.

6. Chirurgische Vorrichtung gemäß Anspruch 5, wobei der Durchgang (131) den ersten Durchmesser definiert, die Zugangsvorrichtung (20) den zweiten Durchmesser definiert und der erste Durchmesser größer als der zweite Durchmesser ist.

7. Chirurgische Vorrichtung gemäß Anspruch 6, wobei die Schnur konfiguriert ist, die Zugangsvorrichtung (20) innerhalb des Durchgangs der flexiblen Hülse (100) zu befestigen.

8. Chirurgische Vorrichtung gemäß einem der vorhergehenden Ansprüche, wobei die flexible Hülse (100) ein proximales Ringelement (111) und ein distales Ringelement (121) aufweist, die an jedem Ende davon befestigt sind.

9. Chirurgische Vorrichtung gemäß Anspruch 8, wobei das distale Ringelement (121) für die Anordnung im Inneren des Gewebetraktes angepasst ist.

10. Chirurgische Vorrichtung gemäß Anspruch 8 oder Anspruch 9, wobei das proximale Ringelement (111) für die Anordnung außerhalb des Gewebetraktes angepasst ist.

11. Chirurgische Vorrichtung gemäß einem der Ansprüche 8 bis 10, wobei:
die Ringelemente (111, 121) entlang der Längsachse (L) angeordnet sind;
die flexible Hülse (100) einen Mantel (130) aufweist, der sich zwischen zwei Ringelementen (111, 121) entlang der Längsachse (L) erstreckt; und
die Schnur (140) zwei Enden aufweist, wobei das eine Ende an einem Ringelement (111, 121) befestigt ist, wobei die Schnur eine ausreichende Länge aufweist, um rund um die Außenfläche des Mantels geschlungen und gebunden zu werden, um den Mantel von einem ersten Durchmesser zu einem zweiten Durchmesser übergehen zu lassen.

12. Chirurgische Vorrichtung gemäß Anspruch 11, wobei der Mantel (130) einen Durchgang (131) definiert, der sich zwischen zwei Ringelementen (111, 121) erstreckt und konfiguriert ist, einen Gegenstand (20) aufzunehmen.

13. Chirurgische Vorrichtung gemäß Anspruch 11 oder Anspruch 12, wobei der Mantel (130) eine im Allgemeinen zylinderförmige Konfiguration definiert.

14. Chirurgische Vorrichtung gemäß einem der vorhergehenden Ansprüche, wobei die Schnur (140) konfiguriert ist, den Durchmesser des Durchgangs (131) zu verringern, um ein abdichtendes Verhältnis mit der Zugangsvorrichtung (20) zu erschaffen.

15. Chirurgische Vorrichtung gemäß einem der vorhergehenden Ansprüche, wobei die komplette Schnur außerhalb des Körperhohlraums angeordnet ist.

16. Chirurgische Vorrichtung gemäß einem der vorhergehenden Ansprüche, wobei die komplette Schnur außerhalb des Gewebetraktes angeordnet ist.

## Revendications

1. Appareil chirurgical à positionner à l'intérieur d'un tractus tissulaire ayant accès à une cavité corporelle sous-jacente comprenant :
un manchon souple (100) définissant un axe longitudinal (L), une extrémité proximale (110), une extrémité distale (120) et un passage (131) pour la réception d'un objet, l'extrémité proximale à localiser à l'extérieur de la cavité corporelle, l'extrémité distale à insérer dans la cavité corporelle, les extrémités proximale et distale étant ouvertes pour fournir un accès à la cavité corporelle, et
un cordon (140) ;
**caractérisé en ce qu'**une extrémité du cordon est attachée à l'extrémité proximale du manchon souple (100), le reste du cordon étant libre, et
l'appareil comprend en outre un dispositif d'accès (20) pouvant être inséré dans le manchon souple (100), de sorte que le cordon (140) est conçu pour être en boucle et serré autour de la surface extérieure dudit manchon souple, réduisant de ce fait le diamètre du manchon souple pour créer une relation d'étanchéité entre ledit dispositif d'accès et ledit manchon.

2. Appareil chirurgical selon la revendication 1, dans lequel le cordon (140) est conçu pour serrer autour d'une surface extérieure (130b) du manchon souple (100).

3. Appareil chirurgical selon la revendication 1, dans lequel le manchon souple (100) inclut une gaine (130) dans une configuration globalement cylindrique.

4. Appareil chirurgical selon l'une quelconque des revendications précédentes, dans lequel l'appareil chirurgical définit une hauteur réglable.

5. Appareil chirurgical selon l'une quelconque des revendications précédentes, dans lequel la formation en boucle et le serrage du cordon (140) autour de la surface extérieure du manchon souple (100) fait subir une transition au manchon souple d'un premier diamètre à un second diamètre.

6. Appareil chirurgical selon la revendication 5, dans lequel le passage (131) définit le premier diamètre, le dispositif d'accès (20) définit le second diamètre, et le premier diamètre est plus grand que le second diamètre.

7. Dispositif chirurgical selon la revendication 6, dans lequel le cordon est configuré pour sécuriser le dispositif d'accès (20) à l'intérieur du passage du manchon souple (100).

8. Appareil chirurgical selon l'une quelconque des revendications précédentes, dans lequel le manchon souple (100) inclut un élément annulaire proximal (111) et un élément annulaire distal (121) attachés à chaque extrémité de ce dernier.

9. Appareil chirurgical selon la revendication 8, dans lequel l'élément annulaire distal (121) est conçu pour être disposé à l'intérieur du tractus tissulaire.

10. Appareil chirurgical selon la revendication 8 ou la revendication 9, dans lequel l'élément annulaire proximal (111) est conçu pour être disposé à l'extérieur du tractus tissulaire.

11. Appareil chirurgical selon l'une quelconque des revendications 8 à 10, dans lequel
les éléments annulaires (111, 121) sont agencés le long de l'axe longitudinal (L) ;
le manchon souple (100) comprend une gaine (130) s'étendant entre les deux éléments annulaires (111, 121) le long de l'axe longitudinal (L) ; et
le cordon (140) a deux extrémités, une extrémité étant attachée à un élément annulaire particulier (111, 121), dans lequel le cordon a la longueur suffisante pour former une boucle et serrer la surface extérieure de la gaine faisant subir une transition à la gaine d'un premier diamètre à un second diamètre.

12. Appareil chirurgical selon la revendication 11, dans lequel la gaine (130) définit un passage (131) s'étendant entre les deux éléments annulaires (111, 121) et configurée pour recevoir un objet (20).

13. Appareil chirurgical selon la revendication 11 ou la revendication 12, dans lequel la gaine (130) définit une configuration globalement cylindrique.

14. Appareil chirurgical selon l'une quelconque des revendications précédentes, dans lequel le cordon (140) est configuré pour réduire le diamètre du passage (131) pour former une relation d'étanchéité avec le dispositif d'accès (20).

15. Appareil chirurgical selon l'une quelconque des revendications précédentes, dans lequel le cordon entier est disposé à l'extérieur de la cavité corporelle.

16. Appareil chirurgical selon l'une quelconque des revendications précédentes, dans lequel le cordon entier est disposé à l'extérieur du tractus tissulaire.
